# EUROPÄISCHE PATENTSCHRIFT

(11) **EP 2 144 636 B1**
(45) Veröffentlichungstag und Bekanntmachung des Hinweises auf die Patenterteilung: **20.06.2012**
(21) Anmeldenummer: 08735166.4
(22) Anmeldetag: 11.04.2008
(51) Int. Cl.: A61L 2/20, B67C 7/00, B65B 55/10

(54) **VERFAHREN ZUM STERILISIEREN VON BEHÄLTERN**
METHOD FOR THE STERILIZATION OF CONTAINERS
PROCÉDÉ DE STÉRILISATION DE CONTENANTS

(30) Priorität: 27.04.2007 DE 102007020458
(43) Veröffentlichungstag der Anmeldung: 20.01.2010
(73) Patentinhaber: KHS GmbH, 44143 Dortmund (DE)
(72) Erfinder: SANGI, Daryoush, 20251 Hamburg (DE); HEROLD, Thomas, 22926 Ahrensburg (DE)
(86) Internationale Anmeldenummer: PCT/EP2008/002858
(87) Internationale Veröffentlichungsnummer: WO 2008/135132

(56) Entgegenhaltungen:
- EP-A- 0 590 505
- WO-A-2007/134803
- DE-A1- 19 949 692
- US-B1- 6 702 985

## Beschreibung

Die Erfindung bezieht sich auf ein Verfahren zum Sterilisieren von Behältern gemäß Oberbegriff Patentanspruch 1.

Verfahren zum Sterilisieren von Flaschen, Dosen oder dergleichen Behältern unter Verwendung eines Wasserstoffperoxyd (H₂O₂) enthaltenden Sterilisationsmedium, d.h. unter Verwendung eines Sterilisationsmediums (nachstehend auch H₂O₂-Sterilisatinsmedium), welches Wasserstoffperoxyd in Mischung mit heißer steriler Luft enthält, sind bekannt. Derartige Verfahren sind beispielweise in US 6,702,985 B1, DE 199 49 692 A1 oder DE 199 49 682 A1 beschrieben.

Bei diesen Verfahren, die z.B. zum Sterilisieren von Behältern für Getränke, aber auch zum Sterilisieren von Behältern oder Verpackungen für andere Produkte, wie z. B. Arzneimittel, verwendet werden, wird beim Einbringen des heißen H₂O₂-Sterilisatinsmedium an der Innenfläche des kühleren Behälters durch Kondensation ein H₂O₂-Kondensationsfilm gebildet, der dann in einer darauffolgenden Aktivierungsphase durch Einbringen eines sterilen heißen gas- und/oder dampfförmigen Aktivierungsmediums, beispielsweise durch Einbringen von heißer steriler Luft in der Weise aktiviert wird, dass durch Zerfall von H₂O₂ freie Sauerstoffradikale entstehen, die zur Sterilisation der Behälter mit vorhandenen Keimen und Verunreinigungen reagieren.

Aufgabe der Erfindung ist es, ein Verfahren und eine Vorrichtung aufzuzeigen, womit unter Aufrechterhaltung einer hohen Entkeimungsrate, d.h. einer hohen Qualität der Sterilisation die Behandlungsdauer insgesamt und dabei speziell auch die Dauer der Aktivierungsphase reduziert werden kann, und zwar bei schonender Behandlung der Behälter. Zur Lösung dieser Aufgabe ist ein Verfahren entsprechend dem Patentanspruch 1 ausgebildet. Die Vorrichtung ist gemäß dem Anspruch 12 ausgebildet, wobei die nachfolgenden abhängigen Ansprüche Ausführungsvarianten aufzeigen.

Mit dem erfindungsgemäßen Verfahren und der Vorrichtung ergibt sich u.a. eine erhebliche Reduzierung der Verfahrensdauer und dabei speziell der Gesamtdauer der Aktivierungsphase, Gleichzeitig erfolgt auch eine schonende und eine thermlrilisation bzw. bei hoher Entkeimungsrate. Das erfindungsgemäße Verfahren eignet sich daher insbesondere für Behälter aus Kunststoff, z.b. aus PET. Weiterbildungen der Erfindung sind Gegenstand der Unteransprüche. Die Erfindung wird im Folgenden im Zusammenhang mit den Figuren an einem Ausführungsbeispiel näher erläutert. Es zeigen:
- Fig. 1: in sehr vereinfachter Darstellung und in Draufsicht eine Maschine bzw. Vorrichtung zum Durchführen des erfindungsgemäßen Verfahrens;
- Fig. 2: in vereinfachter Darstellung einen Behandlungskopf der Vorrichtung der Figur 1.

Die in den Figuren allgemein mit 1 bezeichnete Vorrichtung zur Sterilisierung von Flaschen bzw. das Verfahren zeigen einen um eine vertikale Maschinenachse umlaufend antreibbaren Rotor 2 zur Applikation des Sterilisierungsmittels in die zu behandelnden Flaschen 3, die über einen Behältereinlaufstern 4 zugeführt und aus dem die behandelten, d.h. die benetzten Flaschen 3 über einen Behälterauslauf 5 entnommen und dem nachfolgenden Aktivator zugeführt werden. Der Aktivator ist ebenfalls ein um die vertikale Maschinenachse umlaufend antreibbarer Rotor 6 zur Aktivierung des Sterilisierungsmittels mittels steriler Heizluft, die in die zu behandelnden Flaschen 3 geleitet wird. Die Flaschen 3 werden über einen Behältereinlaufstern 7 dem Rotor 6 zugeführt und die behandelten, d.h. sterilisierten Flaschen 3 über einen Behälterauslauf 8 entnommen und dem nachfolgenden Verfahrensstufe, in der Regel einem Füller, zugeführt.

Oberhalb jeder Flaschenöffnung sind in bekannter Weise am Rotor 2 Applikationsköpfe vorgesehen, welche mit dem Rotor 2 umlaufen und nur als doppelte, gestrichelte Linien I angedeutet sind. Jedem Applikationskopf ist am Rotor 2 ein Flaschen- oder Behälterträger 14 zugeordnet, an dem die jeweilige Flasche 3 während der Behandlung unterhalb des Behandlungskopfes 6 gehalten wird und zwar bei der beispielhaften Ausführungsform, eine als PET-Flaschen ausgebildeten Flaschen 3, an einem flaschenseitigen Mündungsflansch hängend.

Die Benetzung der Oberflächen der Flaschen 3 erfolgt unter Verwendung des H₂O₂-Sterilisationsmedium, welches in bekannter Weise innerhalb des jeweiligen Behandlungskopfes durch Einsprühen von Wasserstoffperoxyd, beispielsweise von 35%-igem Wasserstoffperoxyd in sterile Luft und durch Erhitzen des so erhaltenen Aerosols auf eine Temperatur T₁ von beispielsweise 145°C erwärmt wird.

Für die Behandlung wird heißes H₂O₂-Sterilisationsmedium in das Innere der Flasche 3 eingebracht und zwar derart, dass sich durch Kondensation an der im Vergleich zur Temperatur T₁ des H₂O₂-Sterilisationsmediums kälteren Innenfläche der Flasche 3 ein H₂O₂-Kondensationsfilm bildet, der zumindest die gesamte Innenfläche der jeweiligen Flasche 3 mit einem H₂O₂-Kondensationsfilm gleichmäßig überzieht.

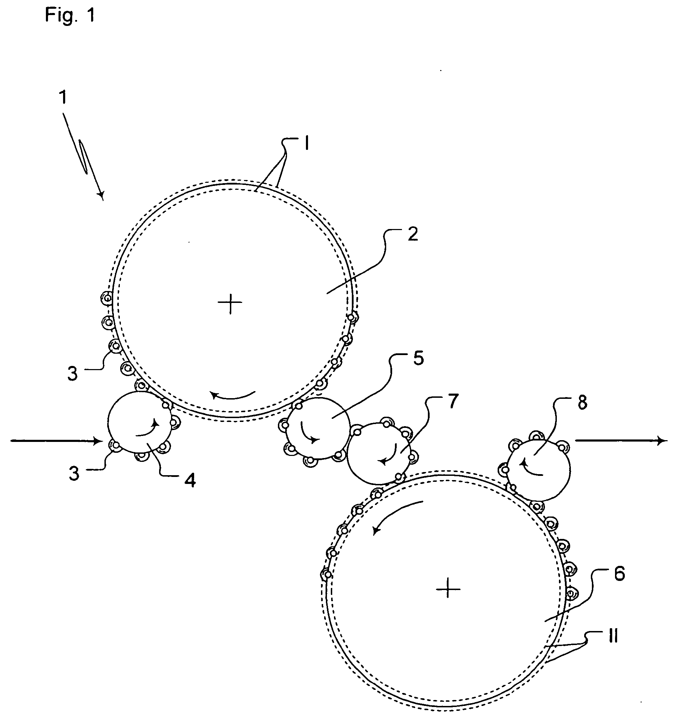

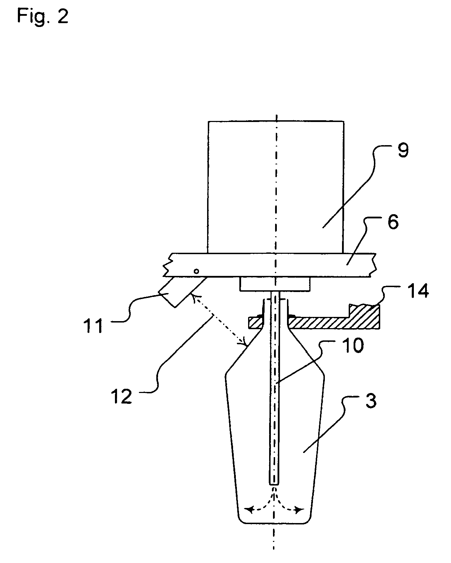

Im Anschluss an diese Applikationsphase und nach der Überleitung der so benetzten Flaschen auf den Rotor 6 erfolgt dann in einer weiteren Behandlungsphase, d.h. in einer Aktivierungsphase, eine Aktivierung des H₂O₂-Kondensationsfilmes. Hierzu sind analog dem Aufbau und Anordnung am Rotor 2 oberhalb jeder Flaschenöffnung in bekannter Weise Aktivatorköpfe 9 (Fig. 2) vorgesehen, welche mit dem Rotor 6 umlaufen und in Fig. 1 lediglich als doppelte, gestrichelte Linien II angedeutet sind. Die Aktivierung wird durch Energieeintrag gestartet und zwar durch Einleitung eines heißen sterilen gas- und/oder dampfförmigen Mediums, durch Einleitung von heißer steriler Luft mit einer Temperatur T₂ in die jeweilige Flasche 3 und zwar durch ein in diese Flasche eingeführtes Rohr 10 (Fig. 2). Mit dieser Aktivierung erfolgt eine Zerfallreaktion von H₂O₂, in deren Verlauf u.a. freie SauerstoffRadikale entstehen, die mit vorhandenen Keimen und/oder Verunreinigungen in der jeweiligen Falsche 3 reagieren und so deren Sterilisation bewirken. Mit der in der Aktivierungsphase verwendeten heißen sterilen Luft erfolgt zugleich auch ein Trocknen der jeweiligen Flasche 3. Sterilisationsverfahren mit diesen Verfahrensschritten sind grundsätzlich bekannt.

Das erfindungsgemäße Verfahren zeichnet sich gegenüber bekannten Verfahren durch eine spezielle Ausgestaltung der einzelnen Behandlungsphasen bzw. deren Verfahrensschritte aus.

In der Applikationsphase erfolgt das Einbringen des heißen H₂O₂-Sterilisationsmediums mit einer konstanten Temperatur T₁ und mit einer konstanten Abgabe- oder Applikationszeit, beispielsweise mit einer Abgabezeit von 3 s bei Flaschen oder Behältern mit einem Volumen von 500 ml. Der Volumenstrom v₁ des in die jeweilige Falsche 3 eingebrachten heißen H₂O₂-Sterilisationsmediums ist dabei beispielsweise während der Applikationszeit ebenfalls konstant.

Das Aktivieren des H₂O₂-Kondensationsfilmes in der jeweiligen Flasche 3 erfolgt während der Aktivierungsphase in zwei Verfahrens-/Aktivierungsschritten. Während eines ersten Verfahrensschrittes wird die zur Aktivierung verwendete heiße sterile Luft mit einer Temperatur T₂ mit einem konstanten, großen Volumenstrom v₂ über das Rohr 10 in die Flasche 3 eingebracht. Dieses Einbringen erfolgt beispielsweise über eine vorgegebene Abgabezeit von x Sekunden oder aber solange, bis die Temperatur der Behälterwandung T_{BW} der jeweiligen Flasche 3 eine vorgegebene Solltemperatur T_{BWsoll} erreicht hat, die über einen Pyrometer 11 (Fig. 2) überwacht und/oder geregelt wird. Der strichpunktierte Pfeil 12 skizziert den Messvorgang. Die Gesamtdauer dieses ersten Verfahrensschrittes ist ca. 4 bis 5 Sekunden.

In einem anschließenden weiteren Verfahrensschritt wird dann das heiße Aktivierungsmedium, welches wiederum heiße sterile Luft ist, mit der Temperatur T₃ und mit einem Volumenstrom v₃ in die Flasche 3 eingebracht, und zwar über eine Abgabezeit von y Sekunden. Der Volumenstrom v₃, der kleiner ist als der Volumenstrom v₂, wird dabei in Abhängigkeit von der Behältertemperatur T_{Bw} der jeweiligen Flasche 3 derart geregelt, dass auch während dieses zweiten Verfahrensschrittes der Aktivierungsphase die Behältertemperatur T_{BW} die Solltemperatur Soll-T_{BW} aufweist. T₃ ist identisch mit T₂ und der Volumenstrom v₃ wird über eine Drossel im Leitungsweg der Sterilluft eingestellt.

Die Solltemperatur Soll-T_{BW} liegt dabei in beiden Verfahrensschritten unter einer Temperatur, die zu einer übermäßigen Belastung oder Verformung oder zu einer Beschädigung der Flaschen 3 führen würde. Da die Temperatur der Flaschenwandung überwacht wird, kann ein steiler Erwärmungsgradient gewählt werden und die Temperatur im zweiten Aktivierungsschritt sehr hoch gehalten werden, was in bekannten Anlagen aus Sicherheitsgründen (Verformung der Flasche) nicht einstellbar war.

Bei Flaschen mit einem Flaschenvolumen von 500ml ergeben sich in der Aktivierungsphase in einem Versuchsstand folgende Volumenströme:

| | |
|---|---|
| v₂ = 9,7Nm³/h | v₃ = 4,2Nm³/h |

Die Behältertemperatur T_{BW} wird auch im zweiten Verfahrensschritt berührungslos unter Verwendung wenigstens eines Pyrometers 11 gemessen, wie dies in der Figur 2 angedeutet ist. Abhängig von der Konstruktion des Rotors 6 und dem dazu gehörigen Aktivatorkopf sowie dem Behältermaterial, können auch andere berührungslose Wärmemesssysteme verwendet werden. Bei der in Fig. 2 gezeigten Variante ist der Pyrometer 11 schwenkbar gelagert, um abhängig von der Flaschen- oder Behältergeometrie optimal ausgerichtet werden zu können.

Die Vorteile des erfindungsgemäßen Verfahrens bestehen u.a. darin, dass die Aktivierung viel heftiger abläuft und somit eine wesentliche Verkürzung der Behandlungsdauer möglich ist, d.h. eine Verkürzung unter 10 s ergibt sich. Insbesondere im größeren Leistungsbereich, d.h. bei höherer Leistung der die Vorrichtung 1 aufweisenden Anlage (Anzahl der verarbeiteten Flaschen 3 je Zeiteinheit), bei welcher bisher ein zusätzlicher, dem Rotor 6 nachgeschalteter zweiter Aktivator erforderlich war, kann nunmehr die Aktivierungsphase allein auf dem Rotor 6 durchgeführt werden oder der Rotor 6 kann einen deutlich geringeren Durchmesser aufweisen, wenn zwei Aktivationsrotoren vorgesehen werden sollen. Dies bedeutet auch, dass bei reduziertem Maschinenaufwand eine wesentliche Erhöhung der Maschinenleistung möglich ist. In besonderen Fällen kann die Applikationsphase und die Aktivierungsphase auf einem einzigen Rotor durchgeführt werden.

Da das erfindungsgemäße Verfahren auf einer selbsttätigen Regelung zumindest des Volumenstromes v₃ im zweiten Verfahrensschritt der Aktivierungsphase beruht, sind für den Betreiber einer Anlage zeitaufwendige Einstellungen oder Versuche zur Erzielung einer optimalen Sterilisation von Flaschen oder dergleichen Behälter nicht erforderlich. Die jeweilige Anlage kann vielmehr anhand von Herstellerangaben, die verschiedene Behälter-Formen und/oder -Materialien berücksichtigen, problemlos eingestellt und betrieben werden, wobei dann die Aktivierungsphase bzw. die dortigen Verfahrensschritte automatisch durch die anlageninterne Regelung durchgeführt bzw. geregelt werden.

Die wesentlichen Parameter einer Ausführungsform des erfindungsgemäßen Verfahrens zum Sterilisieren von Flaschen 3 mit einem Volumen von 500ml lassen sich wie folgt, zusammenfassen:

### Applikationsphase

| | |
|---|---|
| H₂O₂-Konzentration im H₂O₂-Sterilisationsmedium: | 20% |
| maximale Behältertemperatur T_{BW}: | ca. 35°C - 42°C |
| Temperatur T₁: | ca. 145°C |
| Druck des H₂O₂-Sterilisationsmediums: | ca. 0,7 bar |
| Volumenstrom V₁: | ca. 1,5 l/Flasche |
| Volumenstrom v₁: | ca. 2,7 Nm³/h |

### Aktivierungsphase - Verfahrensschritt 1

| | |
|---|---|
| maximale Behältertemperatur T_{BW}: | ca. 67°C - 68°C |
| Volumenstrom v₂: | ca. 10,8 I/Flasche |
| | 9,7 Nm³/h |
| Dampfdruck: | ca. 1,0 bar |
| Luftdruck: | ca. 1,5 bar |

### Aktivierungsphase - Verfahrensschritt 2

| | |
|---|---|
| Behältertemperatur T_{BW}: | ca. 67°C - 68°C |
| Volumenstrom v₃: | ca. 7,0 I/Flasche |
| | ca. 4,2 Nm³/h |

Die Behandlungszeiten der Aktivierungsphasen ist in Summe kleiner als 10 s, wobei die Behandlungszeiten x und y unterschiedlich aber auch gleich sein können. Weiterhin ist es möglich, zwischen der Applikationsphase und der Aktivierungsphase beispielsweise eine Behandlungspause von z.B. ca. 4 bis 5 s vorzusehen, d.h. der erste Verfahrensschritt der Aktivierungsphase wird dann mit einer zeitlichen Verzögerung von beispielsweise ca. 5 sec nach dem Einbringen des H₂O₂-Sterilisationsmediums bzw. nach dem Abschluss der Applikationsphase eingeleitet.

Die Erfindung wurde voranstehend an einem Ausführungsbeispiel beschrieben. Es versteht sich, dass zahlreiche Änderungen sowie Abwandlungen möglich sind, ohne dass dadurch der Erfindung zugrunde liegende Erfindungsgedanke verlassen wird. So wurde voranstehend davon ausgegangen, dass die Behandlungsköpfe 6 Teil einer Behandlungsmaschine oder Vorrichtung umlaufender Bauart sind. Selbstverständlich kann das erfindungsgemäße Verfahren auch auf Anlagen durchgeführt werden, die als Linearmaschinen ausgebildet sind. Weiterhin wurde vorstehend davon ausgegangen, dass das Einbringen des H₂O₂-Sterilisationsmediums und das Einbringen des Aktivierungsmediums jeweils über ein und denselben Behandlungskopf 6 erfolgen. Selbstverständlich können in diesen Verfahrensschritten auch unterschiedliche Behandlungsköpfe zur Anwendungen kommen.

## Patentansprüche

1. **Verfahren zur Sterilisation von Flaschen**, Dosen oder dergleichen Behältern (3) durch Einbringen von dampfförmigen H₂O₂ bzw. eines heißen H₂O₂-Sterilisationsmediums in den jeweiligen Behälter (3) in einer Applikationsphase sowie durch Aktivieren des H₂O₂-Sterilisationsmediums In einer Aktivierungsphase durch Einbringen eines sterilen gas- und/oder dampfförmigen heißen Aktivierungsmediums, durch Einbringen von heißer steriler Luft In den jeweiligen Behälter (4), wobei die Aktivierungsphase wenigstens zwei Aktivierungsschritte aufweist, **dadurch gekennzeichnet, dass**
zumindest während einer Tellphase der Aktivierungsphase der Volumenstrom (v₂, v₃) des In den jeweiligen Behälter (4) eingebrachten Aktivierungsmediums in Abhängigkeit von der Behältertemperatur (T_{BW}) bzw. der Temperatur der Wandung des Behälters (3) geregelt wird, wobei in dem zeitlich letzten Aktivierungsschritt der Volumenstrom (v₃) des dem jeweiligen Behälter (3) zugeführten Aktivierungsmediums in Abhängigkeit von der Behältertemperatur (T_{BW}) geregelt und die die Behältertemperatur (T_{BW}) berührungslos gemessen wird, wobei der Volumenstrom (v₃) der zweiten Aktivierungsphase kleiner als der Volumenstroms (v₂) der ersten Aktivierungsphase ist.

2. Verfahren nach Anspruch 1, **dadurch gekennzeichnet, dass** der Volumenstrom (v₃) der zweiten Aktivierungsphase nur 40% bis 60% des Volumenstroms (v₂) der ersten Aktivierungsphase beträgt.

3. Verfahren nach Anspruch 1 oder 2, **dadurch gekennzeichnet, dass** der Volumenstrom (v₂, v₃) so geregelt wird, dass die Behältertemperatur (T_{BW}) einer Solltemperatur (T_{BWsoll}) entspricht.

4. Verfahren nach Anspruch 1, 2 oder 3, **dadurch gekennzeichnet, dass** die Behältertemperatur (T_{BW}) mit einem Pyrometer gemessen wird.

5. Verfahren nach einem der vorhergehenden Ansprüche, **dadurch gekennzeichnet, dass** in dem zeitlich ersten Verfahrensschritt der Volumenstrom (v₂) des dem jeweiligen Behälter (3) zugeführten Aktivierungsmediums in Abhängigkeit von der Behältertemperatur (T_{BW}) geregelt wird.

6. Verfahren nach einem der vorhergehenden Ansprüche, **dadurch gekennzeichnet, dass** die Aktivierungsphase wenigstens zwei Verfahrensschritte aufweist und dass in dem zeitlich ersten Verfahrensschritt bei konstanter Temperatur (T₂) oder bei im Wesentlichen konstanter Temperatur und bei konstantem oder im Wesentlichen konstanten Volumenstrom (v₂) das Zuführen des Aktivierungsmediums in den jeweiligen Behälter (3) zeitgesteuert erfolgt.

7. Verfahren nach einem der vorhergehenden Ansprüche, **dadurch gekennzeichnet, dass** dem Jeweiligen Behälter (3) in der Applikationsphase das heiße Sterilisationsmedium mit konstanter oder im Wesentlichen konstanter Temperatur (T₁) zeitgesteuert zugeführt wird.

8. Verfahren nach einem der vorhergehenden Ansprüche, **dadurch gekennzeichnet, dass** während der Applikationsphase das heiße Sterilisationsmedium dem jeweiligen Behälter (4) mit konstanter oder im Wesentlichen konstanter Temperatur und über eine konstante oder im Wesentlichen konstante Zeitdauer mit einem Volumenstrom (v₁) zugeführt wird, der unter Berücksichtigung der Behältertemperatur (T_{BW}) derart abgestuft bzw. gesteuert ist, dass die Behältertemperatur (T_{BW}) deutlich unter der Temperatur (T₁) des heißen H₂O₂-Sterilisationsmediums bleibt.

9. Verfahren nach einem der vorhergehenden Ansprüche, **dadurch gekennzeichnet, dass** während der Applikationsphase das Zuführen des heißen H₂O₂-Sterilisatlonsmedlums mit einer Applikationsdauer von 2,5 bis 4 sec. erfolgt.

10. Verfahren nach einem der vorhergehenden Ansprüche, dadurch gekennzelchnet, dass die Behandlungsdauer eines Aktivierungsschrittes kleiner als 10 Sekunden ist.

11. Verfahren nach einem der vorhergehenden Ansprüche, **dadurch gekennzeichnet, dass** die Aktivierungsphase auf einen einzigen Aktivator durchgeführt wird.

12. **Sterillsierungsvorrichtung** für Behälter, wie Flaschen, Becher, Dosen und dergleichen zur Durchführung des Verfahrens nach einem der Ansprüche 1 bis 11, **dadurch gekennzeichnet, dass**
diese mindestens eine Vorrichtung zur berührungslosen Temperaturmessung von Festkörperoberflächen und eine hiermit verbundene computergestützte Steuer- und Regeleinrichtung zur Messwertauswertung sowie zur Temperatur- und/oder Volumenstromregelung aufweist, wobei die Schritte der mindestens zwei Aktivierungsphasen von einander unabhängig und auf einer einzigen Transportvorrichtung durchführbar sind, indem zur Regelung des Volumenstroms der Aktivierungsmedien in jedem Leitungsweg eines Aktivierungsmediums zu den jeweiligen Aktivatorkopf (9) mindestens eine Drossel, ein Ventil oder dergleichen angeordnet ist, wobei an oder im Nahbereich mindestens einer Teilzahl der Aktivatorköpfe (9) Vorrichtungen zur berührungslosen Temperaturmessung von Festkörperoberflächen angeordnet sind, wobei an oder im Nahbereich eines jeden Aktivatorkopfes (9) eine Vorrichtung zur berührungslosen Temperaturmessung von Festkörperoberflächen angeordnet ist..

13. Sterilisierungsvorrichtung gemäß Anspruch 12, **dadurch gekennzeichnet, dass** die mindestens eine Vorrichtung zur berührungslosen Temperaturmessung von Festkörperoberflächen ein Pyrometer ist.

## Claims

1. **A method for sterilizing bottles**, cans or similar vessels (3) by introducing either vaporous H₂O₂ or a hot H₂O₂ sterilization medium in a particular vessel (3) in an application phase, as well as by activating the H₂O₂ sterilization medium in an activation phase by introducing a sterile gaseous and/or vaporous activation medium and by introducing hot, sterile air in a particular vessel (4), where the activation phase features two procedural steps, **characterised in that,**
at least during part of the activation phase the volume flow rate (v₂, v₃) of the activation medium introduced into the particular vessel (4) is regulated, depending on either the vessel temperature (T_{BW}) or the temperature of the wall of the vessel (3), where the volume flow rate (v₃) of the activation medium directed into the particular vessel is regulated, depending on the vessel temperature (T_{BW}), in the chronologically last activation step, and that the vessel temperature (T_{BW}) is measured with no contact, where the volume flow rate (v₃) in the second activation phase is less than the volume flow rate (v₂) in the first activation phase.

2. The method of claim 1, **characterised in that** the volume flow rate (v₃) in the second activation phase amounts to only 40-60% of the volume flow rate (v₂) in the first activation phase.

3. The method of claim 1 or 2, **characterised in that** the volume flow rate (v₂, v₃) is regulated so the vessel temperature (T_{BW}) corresponds to the set-point temperature (T_{BWsoll}).

4. The method of claim 1, 2 or 3, **characterised in that** the vessel temperature (T_{BW}) is measured with a pyrometer.

5. The method of any one of the preceding claims, **characterised in that** the volume flow rate (v₂) of the activation medium directed into the particular vessel (3) is regulated in the chronologically first procedural step, depending on the vessel temperature (T_{BW}).

6. The method of any one of the preceding claims, **characterised in that** the activation phase features two procedural steps and the activation medium is directed to the particular vessel (3) in a time-controlled manner during the chronologically first step at a constant temperature (T₂) or at a roughly constant temperature and at a constant or roughly constant volume flow rate (v₂).

7. The method of any one of the preceding claims, **characterised in that** the hot sterilization medium is directed to the particular vessel (3) in a time-controlled manner at a constant or roughly constant temperature (T₁).

8. The method of any one of the preceding claims, **characterised in that** the hot sterilization medium is directed during the application phase into the particular vessel (4) at a constant or roughly constant temperature and over a constant or roughly constant period of time at a volume flow rate (V₁) that, taking into account the vessel temperature (T_{BW}), is so staged or driven that the vessel temperature (T_{BW}) remains well below the temperature (T₁) of the hot H₂O₂ sterilization medium.

9. The method of any one of the preceding claims, **characterised in that** the hot H₂O₂ sterilization medium is fed during the application phase with application lasting 2.5 - 4 seconds.

10. The method of any one of the preceding claims, **characterised in that** the duration of treatment in an activation step is less than 10 seconds.

11. The method of any one of the preceding claims, **characterised in that** the activation phase is carried out on a single activator.

12. **A sterilization device** for vessels such as bottles, mugs, cans and the like for conducting the method in any of the claims 1-11, **characterised in that**, at least a device for no-contact measurement of solid surface temperatures and a computerized control and regulating device connected therewith to regulate temperature and/or volume flow rate is shown, where the steps in at least two activation phases are independent of each other and are feasible on a single transport device, while at least a throttle, a valve or the like is arranged to regulate the volume flow rate of the activation medium in routing the activation medium to an activator head (9), where devices for no-contact measurement of solid surface temperatures are arranged on or near at least a fraction of the activator head (9) and where a device for no-contact measurement of solid surface temperatures is arranged on or in the vicinity of the activator head (9).

13. The sterilization device according to claim 12, **characterised in that** at least one device for no-contact measurement of solid surface temperatures is a pyrometer.

## Revendications

1. Procédé de stérilisation de bouteilles, de boîtes ou conteneurs similaires (3) par insertion de H₂O₂ sous forme de vapeur ou de milieu de stérilisation de H₂O₂ chaud dans le conteneur respectif (3) dans une phase d'application et par activation du milieu de stérilisation de H₂O₂ dans une phase d'activation par insertion d'un milieu d'activation stérile chaud sous forme de gaz et/ou de vapeur, par insertion d'air stérile chaud dans le conteneur respectif (4), la phase d'activation présentant au moins deux étapes d'activation, **caractérisé en ce que** au moins pendant une phase partielle de la phase d'activation, le courant volumique (V₂, V₃) du milieu d'activation inséré dans le conteneur respectif (4) est régulé en fonction de la température du conteneur (T_{BW}) ou de la température de la paroi du conteneur (3), dans dernière étape d'activation, d'un point de vue temporel, le courant volumique (V₃) du milieu d'activation acheminé dans le conteneur respectif (3) étant régulé en fonction de la température du conteneur (T_{BW}) et la température du conteneur (T_{BW}) étant mesurée sans contact, le courant volumique (V₃) de la deuxième phase d'activation étant inférieur au courant volumique (V₂) de la première phase d'activation.

2. Procédé selon la revendication 1, **caractérisé en ce que** le courant volumique (V₃) de la deuxième phase d'activation n'est que de 40 % à 60 % du courant volumique (V₂) de la première phase d'activation.

3. Procédé selon la revendication 1 ou 2, **caractérisé en ce que** le courant volumique (V₂, V₃) est régulé de telle sorte que la température du conteneur (T_{BW}) corresponde à une température théorique (T_{BWsoll}).

4. Procédé selon la revendication 1, 2 ou 3, **caractérisé en ce que** la température du conteneur (T_{BW}) est mesurée avec un pyromètre.

5. Procédé selon l'une des revendications précédentes, **caractérisé en ce que** dans la première étape du procédé, d'un point de vue temporel, le courant volumique (V₂) du milieu d'activation acheminé au conteneur respectif (3) est régulé en fonction de la température du conteneur (T_{BW}).

6. Procédé selon l'une des revendications précédentes, **caractérisé en ce que** la phase d'activation présente au moins deux étapes du procédé et **en ce que**, dans la première étape du procédé, d'un point de vue temporel, l'acheminement du milieu d'activation dans le conteneur respectif (3) s'effectue de façon contrôlée dans le temps, à température constante (T₂) ou à une température essentiellement constante et à un courant volumique (V₂) constant ou essentiellement constant.

7. Procédé selon l'une des revendications précédentes, **caractérisé en ce que** le milieu de stérilisation chaud est acheminé au conteneur respectif (3) dans la phase d'application, de façon contrôlée dans le temps, à une température (T₁) constante ou essentiellement constante.

8. Procédé selon l'une des revendications précédentes, **caractérisé en ce que**, pendant la phase d'application, le milieu de stérilisation chaud est acheminé au conteneur respectif (4) à une température constante ou essentiellement constante et pendant une durée constante ou essentiellement constante, à un courant volumique (V₁) qui est gradué ou commandé en tenant compte de la température du conteneur (TBW) de telle sorte que la température du conteneur (T_{BW}) reste nettement inférieure à la température (T₁) du milieu de stérilisation de H₂O₂ chaud.

9. Procédé selon l'une des revendications précédentes, **caractérisé en ce que**, pendant la phase d'application, l'acheminement du milieu de stérilisation de H₂O₂ chaud s'effectue avec une durée d'application de 2,5 à 4 sec.

10. Procédé selon l'une des revendications précédentes, **caractérisé en ce que** la durée du traitement d'une étape d'activation est inférieure à 10 secondes.

11. Procédé selon l'une des revendications précédentes, **caractérisé en ce que** la phase d'activation est réalisée sur un activateur individuel.

12. Dispositif de stérilisation pour conteneurs tels que bouteilles, béchers, boîtes et similaires, pour la réalisation du procédé selon l'une des revendications 1 à 11, **caractérisé en ce**
**qu'**il présente au moins un dispositif de mesure sans contact de la température de surfaces de solides et un dispositif de commande et de régulation assisté par ordinateur, lié à celui-ci, pour évaluer la mesure et pour réguler la température et/ou le courant volumique indépendamment l'un de l'autre, les étapes d'au moins deux phases d'activation pouvant être réalisées indépendamment l'une de l'autre et sur un dispositif de transport unique, par l'aménagement d'au moins un papillon, une soupape ou similaire pour la régulation du courant volumique des milieux d'activation dans la conduite respective d'un milieu d'activation à la tête de l'activateur respectif (9), des dispositifs de mesure sans contact de la température de surfaces de solides étant aménagés sur ou à proximité d'au moins un nombre partiel de têtes d'activateur (9), un dispositif de mesure sans contact de la température de surfaces de solides étant aménagé sur ou à proximité de chacune des têtes d'activateur (9).

13. Dispositif de stérilisation selon la revendication 12, **caractérisé en ce qu'**au moins un dispositif de mesure sans contact de la température de surfaces solides est un pyromètre.
